# EUROPEAN PATENT APPLICATION

(11) **EP 4 270 404 A1**
(43) Date of publication of application: **01.11.2023**
(21) Application number: 22170577.5
(22) Date of filing: 28.04.2022
(51) Int. Cl.: G16H 10/60, G16H 70/20

(54) **MONITORING PATIENT DATA**

(71) Applicant: Koninklijke Philips N.V., 5656 AG Eindhoven (NL)
(72) Inventor: BULUT, Murtaza, Eindhoven (NL); JOHNSON, Mark, Thomas, Eindhoven (NL); CHAKRABARTI, Biswaroop, Eindhoven (NL); BOUWMAN, Wilbert, Hendrik, Eindhoven (NL)
(74) Representative: Philips Intellectual Property & Standards

(57) **Abstract**

The invention relates to a computer-implemented method (600) of monitoring patient data. A patient record data source enables to access a patient record of a patient, wherein the patient record indicates a version of a medical protocol currently being used with the patient. A protocol data source indicates available versions of the medical protocol and/or of a medical guideline from which the medical protocol is derived. The used version of the medical protocol is compared to a further version of the medical protocol or of the medical guideline accessible via the protocol data source to obtain a change indicator for the used version of the medical protocol, and the change indicator is output.

## Description

### FIELD OF THE INVENTION

The invention relates to a computer-implemented method of monitoring patient data, and to a corresponding system. The invention further relates to a computer-readable medium.

### BACKGROUND OF THE INVENTION

Medical procedures are typically performed according to guidelines and protocols derived from them. Guidelines are also known as clinical practice guidelines (CPGs). Generally, a guideline may be a prescription of a treatment of a medical condition that is for use by multiple healthcare providers. Guidelines are typically produced by an organization independent from the healthcare providers that are to use it. Protocols may also be referred to as local guidelines or care pathways.

Development and update of clinical practice guidelines is typically performed by specific organizations. For example, National Comprehensive Cancer Network (NCCN) in USA, prepares guidelines covering 97% of the cancers. The same also applies to Europe. For instance, the European Association of Urology (EAU) prepares guidelines for prostate cancer. Clinical practice guideline preparation is a complicated and continuous process.

For example, the NCCN follows a process for the concurrent development and production of NCCN Guidelines derivative products that uses as inputs: institutional review; clinical data review; PubMed literature review; third-party submissions; scientific meetings/congress presentations; and recent FDA approvals. A panel meeting is held to discuss institutional review comments, relevant literature and submissions, and to vote as needed for changes. Algorithms, manuscripts, and references are updated; and reviewed by the panel chair/vice-chair, and the full panel. Updated guidelines are published, after which the process is repeated. When important new data is released, an interim panel meeting may be held leading to an update of the guidelines.

Generally, once a guideline is finalized, it is disseminated, and further updates may be done based on its application in the field, where both physicians' and patients' input can be considered. A process for guideline development, dissemination, and updating is discussed in D. A. Davis et al., "Translating guidelines into practice. A systematic review of theoretic concepts, practical experience and research evidence in the adoption of clinical practice guidelines", PubMed ID 9275952.

In addition to guideline documents, bodies such as the NCCN and EAU produce documents indicating specifically changes in the guidelines and short versions of guidelines to help the dissemination. In addition, the requests originating from clinical studies, and the decisions taken are also communicated.

The guidelines are processed by healthcare providers and translated to protocols. A protocol may prescribe a way of diagnosing, managing, or treating a patient at a healthcare provider or at a group of multiple collaborating healthcare providers. A protocol may be specific to one or more specific healthcare providers, and typically results from physicians interpreting the guidelines and deciding how they will be applied in practice by the corresponding healthcare provider. Guidelines can be updated several times in a year (e.g., 4 times). Protocols are typically easier and faster to update, and may thus be updated more often than the underlying guidelines, e.g., depending on the preferences of the practicing physicians. A protocol is typically more concrete and streamlined than its parent CPGs, and may be influenced e.g. by resource availability and staff competence. Due to the interpretation process, there can be (sometimes very significant) differences among healthcare providers with respect to local procedures and protocols. Protocols are not always derived from a guideline, e.g., in situations where no guidelines for a particular medical condition are available yet.

Oncology is an example of a medical field that is fast-paced, with new treatment options continuously becoming available. Accordingly, it is desirable to keep patients, for example cancer patients in the case of oncology, as well as their clinicians or their caregivers aware of the newest treatment options relevant for them.

### SUMMARY OF THE INVENTION

In accordance with a first aspect of the invention, a computer-implemented method of monitoring patient data and a corresponding system are provided, as defined by claims 1 and 14, respectively. In accordance with an aspect of the invention, a computer-readable medium is described as defined by claim 15.

Various measures discussed herein relate to the monitoring of patient data. The patient data may be monitored in order to inform stakeholder(s) in the handling (e.g., treatment, management, or diagnosis) of the patient, e.g., a cancer patient, about availability of new options to perform the handling, e.g., the treatment. The stakeholders may be one or more of: the patient, a physician involved in the treatment of the patient, or a non-medical-professional involved in the treatment of the patient, such as a family member, friend, or caregiver.

One way to identify new options may be to identify relevant literature and clinical trials using patient data in a "greedy search"-type method. The inventors realized that such a method has several possible drawbacks, including a need to access detailed patient data, and relatively high cost in terms to computational resources. Further, it is hard for the patient themselves to follow such a route as understanding such specialized searching generally requires their clinician to be involved.

Interestingly, the inventors found a different way to monitor the patient data of a patient in order to enable the signaling of possible availability of new options. Namely, the monitoring may be based on information in a patient record of the patient that indicates a version of a medical protocol that is used with the patient, for example, to guide diagnosis, management, and/or treatment of the patient. For example, the protocol may be currently being used, or it may have been used in the past. For example, the protocol may be a treatment protocol that is used in a current treatment, or in a treatment that has taken place in the past. Such a treatment may be a regular treatment, but can also be in the context of treatment in a medical trial.

The used version may be accessed from a patient record data source, e.g., a personal health record (PHR) or electronic medical record (EMR). This used version of the medical protocol may be compared to a further version, either of the medical protocol itself, or of a medical guideline from which the medical protocol is derived. For example, the further version may be the most recent version of the medical protocol and/or the medical guideline available in the protocol data source. This further version may be accessed from a protocol data source that indicates versions of the protocol and/or guideline that are available, meaning that they may potentially be used for the patient. The comparison may result in a change indicator for the used version of the medical protocol. The change indicator may be output, e.g., to the patient, physician, or other user; or to the patient record for future use.

By comparing the used version to another version, the change indicator may be indicative of protocol/guideline changes with respect to the used protocol, and thus of the possible availability of new options to treat, diagnose, or manage the patient. In a relatively simple embodiment, already the fact that the monitoring detects that a newer version of the protocol or guideline is available, may be a useful change indicator for triggering a manual or automatic determination whether to switch to the newer version of the protocol or guideline. In more advanced embodiments, various protocol change features, such as change count or frequency, may be output as indicators and used for example to start searching if new options are available. Accordingly, the provided techniques may help patients, their caregivers, and their physicians become aware of the changes in options relevant for them. Comparing to a guideline version, e.g., the latest final or draft guideline version, is particularly beneficial: in this way, differences can be identified earlier. This is because translating guidelines to protocols typically takes time. The patient that is monitored may be a cancer patient, but the provided techniques can be applied to other patient groups too, e.g., the patient may be a patient suffering from other diseases than cancer, e.g., a chronic condition or an auto-immune disease.

The "monitoring" of the patient data may refer to a determination of the change indicator that does not take place in response to a direct user command, but is e.g. performed at regular intervals or by being automatically triggered, e.g., by a change in the underlying data. By monitoring, and in various embodiments also quantifying, the changes in the relevant local hospital protocols, a technique is provided to provide information about possible new options, e.g., for treatment or diagnosis, that is not only effective, but that is also computationally efficient and that requires relatively little access to privacy-sensitive patient data. In other words, a change indicator may be determined in an efficient way that facilitates the search for new options by informing physicians, patients, or others involved.

In particular, the use of protocols and/or guidelines for the monitoring per se may lead to improved computational efficiency. In particular, the results of a comparison of a used version of the protocol with further versions may be re-used among patients for which the same version is used, thus saving computational resources. Such reuse is not possible when identifying options based on patient data, that differs among patients. Moreover, during the monitoring, it may be detected that the used version of the medical protocol and the available protocols and/or guidelines have not changed, in which case a more detailed and thus more computationally expensive check may be skipped. Or, the comparison may be initiated in response to a change in the used version of the protocol and/or the available protocols and/or guidelines. In any case, since information about protocols typically does not change frequently, e.g., much less frequently than other patient data, it may often be possible to skip the more computationally more expensive parts of the monitoring, further increasing efficiency.

However, also the comparison between the used and further versions may be carried out in a computationally efficient way. For example, the protocols may be represented as textual data, allowing changes in these text documents to be identified in an efficient manner, for example using text comparison tools as known per se. The identified changes can be processed to extract one or more features, which may for example be used to determine whether to automatically alert the clinician, patient, or other user, about the potential for further options e.g. for treatment or diagnosis. Also such feature extraction may be possible using limited computational power.

In terms of privacy and data minimization of patient data, it may be much more desirable to just use information about the used medical protocol than to use detailed further medical information of the patient. Identification of changes in protocols and change indicator determination may be performed without access to patient data. Based on this identification, for example, a physician may be notified on a per-patient basis, so that the physician can inspect the patient data to see if the protocol/guideline changes are relevant for the patient. Thus the notification of the physician may be performed without using other patient information than the identification of the used protocol version. In fact, it may not even be needed to access contents of the medical protocols or guidelines from the protocol data source, e.g., in case the comparison is based on a timing of updates of the protocol. This is also advantageous because the results of such a comparison may be easy to understand for non-clinicians, e.g., patients, allowing them e.g. to initiate searches and discussions for the latest relevant clinical trials.

In other cases, upon detecting a change with respect to the used protocol, patient data may be used to estimate an applicability of the change to the patient. Also in such cases, privacy is improved since this patent data is accessed in response to an actual change, and only patient data related to the change may be needed. For the same reason, also computation and data transfer and storage needs may be reduced. In yet other cases, when determining an aggregate over multiple patients, the collection of aggregated and/or anonymized information about used versions may be performed separately from the comparison to further protocol/guideline versions, further improving data minimization.

In general, the protocols and/or guidelines may be defined for a particular medical condition. The medical condition can be from the field of oncology, for example. However, the techniques are also applicable to other fields of medicine, e.g., other domains where treatment requires longer time such as a psychological disorder or a depression. For example, the treatment may take at least a week or at least a month.

Optionally, the change indicator may comprise a comparison output for the current version with respect to the further version, and a reference to this further version, e.g., the further guideline version of the further protocol version. The reference may allow to retrieve the further version from the protocol data source, e.g., may comprise a version number, document ID, or other identifier of the guideline or protocol version. The protocol data source can for example be a version control system, in which case the used version and/or the further version to which the used version is compared, may be indicated by a version number according to the version control system.

Generally, a medical protocol or a medical guideline may be stored in the protocol data source as textual data, as structured data, or as a rule set. Typically, protocols have a decision tree structure comprising multiple steps. A step may comprise a particular procedure to be executed, for example, a standard operating procedure, or any other activity. The step may further comprise a decision to be taken based on the outcome of the procedure. The textual data may comprise metadata representing the decision tree organization of the protocol or guideline into the steps, for example, by means of structured data (e.g., according to a defined schema, in a format such as JSON, XML, or similar), or as a rule set (e.g., wherein a rule may indicate premises under which a decision is to be taken). The use of such metadata has the advantage that it makes comparison more computationally efficient and less error-prone. However, comparison may also be based on a textual data representation. Typically, in many cases, the comparison uses the contents of the protocol or guideline, but some cases the comparison may not involve the contents of the medical protocol or guideline at all.

The change indicator may be output in various ways. Optionally, the change indicator may be output to the patient record. This allows to use the change indicator for later automated or manual analysis. The change indicator may also be output by automatically alerting one or more users depending on the output change indicator. The user(s) can for example be the patient; a healthcare provider; a physician involved in the treatment of the patient; a family member; a friend; a caregiver; or more generally a person involved with the care of the patient; and/or an insurance provider. This way the user can be automatically triggered to further investigate options, ultimately allowing the handling, e.g., treatment or diagnosis, of the patient to be improved.

Optionally, the patient record may comprise a user defined function that is configured to automatically alert the user, e.g., the patient and/or the physician. The user-defined function may be physician-defined, for example. This allows the physician themselves to control when to receive change indicators. The user-defined function may be executable by the patient record data source. Thus, the monitoring and the alerting may be performed by separate components, where the patient record data source does not need to be aware of the specifics of the comparison and the monitoring does not need to have alert capabilities. The patient record may comprise multiple user-defined functions relating to the change indicator, e.g., for respective parties and/or for alerts in different circumstances.

Optionally, the used version may be compared to the further version by determining a count and/or timing of changes. For example, the comparison may use a frequency of changes, such as a number of times per period that the protocol is updated per se, or a number of changes per time period. Such high-level features can in some cases be used instead of using the textual contents of the guidelines or protocols. This allows to provide change indicators that can be interpreted also by non-clinicians, e.g., patients, who can thus also benefit from the solution. This is particularly useful for empowering patients to initiate searches and discussions for the latest relevant clinical trials.

Optionally, the comparison may be based on determining comparison outputs for respective procedures of the protocols/guidelines. Such a comparison output may indicate a change between the used version and the further version with respect to application of the given procedure. For example, a comparison output may indicate one of at least: no change or change; or a comparison outputs may indicate one of at least: no change, edit, addition, removal, or rearrangement. A comparison output for an edit may be based on an estimate of the significance of the chance, e.g., a high significance may be attached to a change in vital parameter measurement and/or drug administration (e.g., drug type, time or dosage). The respective comparison outputs may be aggregated into an overall output which may be comprised in, or otherwise used to determine, the change indicator, for example, an overall change count.

In practice, guidelines/protocol updates are relatively small, e.g., an update may involve only edits for a limited number of particular procedures. In such cases, a comparison based on comparison outputs is particularly effective. The comparing may comprise matching data about a procedure between the current and further version and using the matching to determine the comparison outputs; or, outputting a particular change indicator if no matching could be made. If no matching can be made, this is by itself a useful output since it indicates that major differences likely exist.

Optionally, recommendation strength indicators may be obtained for respective procedures of the protocols/guidelines being compared. A recommendation strength indicator may indicate an importance of a guideline change as indicated by the publisher of the guidelines. For example, a recommendation strength indicator may be derived from a NCCN publication, from notes or a vote of a panel discission, and/or from clinical trial results leading to the change, for example, manually or using machine learning. The change indicator may be based on the recommendation strength indicators, e.g., by taking a weighted combination of comparison outputs for respective procedures according to their recommendation strength indicators; by inputting the recommendation strength indicators and comparison outputs to a machine learning algorithm that determines the change indicator, or the like.

Optionally, patient data of the patient may be accessed, and used to determine applicability data indicating an applicability of guideline/protocol changes to the patient. The applicability data may be used to determine the change indicator, and in particular, in case the change indicator is determined from comparison outputs, applicability data for a particular procedure may be used to determine a comparison output for that procedure. Thus, a change indicator more tied to the particular patient may be determined. Still, data minimization may be satisfied since only patient data relating to changes may be used.

Optionally, a feature extractor may be used to obtain a feature extractor output for a given procedure, which may then be used to determine the change indicator. The feature extractor may be applied to data about the given procedure comprised in the used version and/or in the further version, in which case the resulting feature vectors may be compared to each other. It is also possible to apply the feature extractor to a difference between the used version and the further version. The use of feature extractors allows a comparison to be performed efficiently based on feature extraction techniques, e.g., techniques for extracting feature vectors from text, that are known per se.

Optionally, a feature extractor may be applied to the overall protocol/guideline versions being compared, e.g., instead of extracting features per procedure. Also in this case, the feature extractor may be applied to the used version and/or the further version separately, in which case the resulting feature vectors may for example be compared to each other; or, the feature extractor may be applied to a difference between the used version and the further version. In any case, the overall feature extractor output obtained in this way may be used to determine the change indicator. By using an overall feature extractor, a need to separately divide the protocols/guidelines into procedure steps is alleviated and this task may instead be performed implicitly by the feature extractor. This may work particularly well if the amount of change is large.

Optionally, based on the comparison, it may be determined if a clinical trial recommendation applies. If this is the case, the clinical trial recommendation may be output. This can for example enable patients to find clinical trials which can be relevant for them. For example, the clinical trial may be recommended based on predicting a treatment trajectory of the patient according to the further protocol/guideline version; and determining if the further version indicates a clinical trial recommendation for the predicted trajectory. For example, the further version may be annotated with particular clinical trial recommendations for particular predicted trajectories, e.g., for particular nodes of a decision tree representing the guideline or protocol. It is also possible to derive the clinical trial recommendation by other means. For example, a high frequency of change in the protocols/guidelines may be used as an indication that a clinical trial may be ongoing. For this, interestingly, no specific patient information is needed. The clinical trial recommendation may provide a general suggestion to look for a clinical trial but can also indicate a specific clinical trial, for example, by accessing a clinical trial registry and searching for a clinical trial matching the clinical trial recommendation and/or the patient data.

Optionally, the protocol version used for the patient, and the further version against which the used protocol version is compared, may be of different medical institutions and/or from different data sources. Guidelines can be different from one hospital to another, especially when hospital-specific adaptations are made and stored in SOPs which are based on the national guidelines. When the patient moves from one hospital to another, protocols of the new hospital can be different. In case of a transfer from one medical institution to another, comparison of the used protocols may be particularly important. The differences indicated by the change indicator can for example be evaluated by a treating physician, who may determine if the differences require actions, e.g., adaptation of dose, or adoption of protocol changes from the previous hospital if improved outcomes are reported.

Optionally, respective change indicators with respect to a further version may be determined for multiple patients, and an aggregate may be computed over the respective change indicators and output. For example, a guideline update can potentially affect many patients' treatment. Providing an aggregate of the comparisons is more useful to the physician and can help better planning of care. For example, the determination of the aggregate may be triggered by an update at the protocol data source. This can for example enable a physician to view a total number of affected patients and help the physician and hospital to plan accordingly.

Optionally, the comparing may comprise determining a mismatch between versions of multiple medical guidelines from which the medical protocol is derived, and notifying a user of the mismatch. For example, a new version of a first medical guideline may conflict with the latest version of a second medical guideline, e.g., coming from a different organization. The comparison may for instance be triggered by the new version of the first medical guideline. The comparison may compare the used version of the medical protocol to both guidelines and may establish the mismatch, e.g., the used version may comprise a procedure according to the second medical guideline that is not compatible with the first medical guideline, e.g., that uses a significantly different threshold value, that recommends a different type of medicine, etc. In case a conflict is detected, a treating physician and/or a hospital/appropriate authority level expert panel may for example be alerted to avoid poor quality/nonstandard patient management.

It will be appreciated by those skilled in the art that two or more of the above-mentioned embodiments, implementations, and/or optional aspects of the invention may be combined in any way deemed useful. Modifications and variations of any system and/or any computer readable medium, which correspond to the described modifications and variations of a corresponding computer-implemented method, can be carried out by a person skilled in the art on the basis of the present description.

### BRIEF DESCRIPTION OF THE DRAWINGS

These and other aspects of the invention will be apparent from and elucidated further with reference to the embodiments described by way of example in the following description and with reference to the accompanying drawings, in which:
Fig. 1 shows a system for monitoring patient data;
Fig. 2 shows a detailed example of how to determine a change indicator;
Figs. 3a-3b show examples of medical guidelines;
Figs. 4a-4d shows an example of determining change features;
Fig. 5 shows a detailed example of a method of monitoring patient data;
Fig. 6 shows a computer-implemented method of monitoring patient data;
Fig. 7 shows a computer-readable medium comprising data.

It should be noted that the figures are purely diagrammatic and not drawn to scale. In the figures, elements which correspond to elements already described may have the same reference numerals.

### DETAILED DESCRIPTION OF EMBODIMENTS

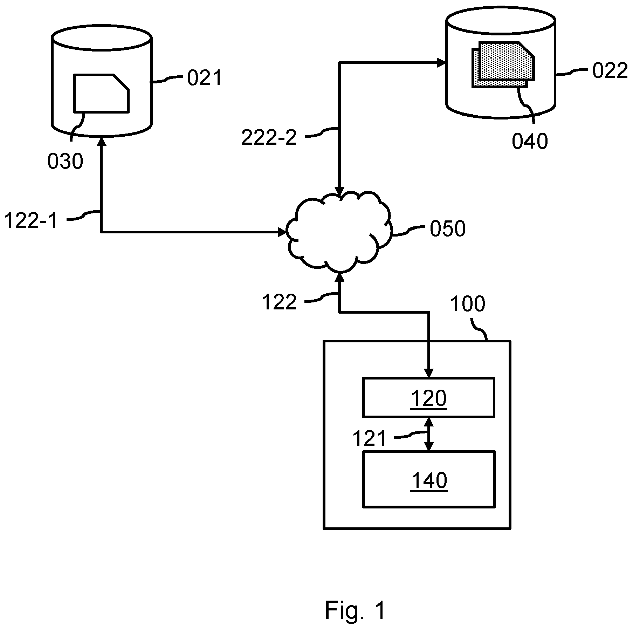

Fig. 1 shows a medical data system 100. System 100 may be for monitoring patient data of a patient. The system may be for operation in a hospital environment and may for example accordingly have direct access to a protocol data source of a hospital. The system can also be for operation outside of a hospital environment, e.g., the system may be a system for maintaining personal health records (PHRs).

The system 100 may comprise a data interface for accessing a patient record data source 021. The patient record data source 021 may enable to access a patient record 030 of a patient. The patient record may indicate a version of a medical protocol used with the patient. The patient record may contain the contents of the protocol version as well, or may link to the protocol version, e.g., the version indication in the patient record may allow these contents to be retrieved from a protocol data source. The patient record may contain further patient data about the patient, as is known per se. The patient record data source 021 may provide access to records of multiple patients, e.g., at least 100, at least 1000, or at least 10000 patient records.

The patient record data source 021 may be managed by a medical institution, e.g., the patient records can be electronic health records. The patient record data source 021 can also be managed by a third party, e.g., the patient records can be personal health records. Such a personal heath record may comprise medical data from multiple health care providers and/or from one or more additional data sources apart from medical institutions, e.g., from one or more wearable sensors. The electronic medical records and/or personal health records may be distributed records, in which case the patient record data source may be distributed as well. For example, a distributed record may be accessed from a distributed ledger, such as a blockchain. As another example, a non-centralized record may be accessed from a database using database access techniques that are known per se.

The system 100 may further comprise a data interface for accessing a protocol data source 022. The protocol data source 022 may indicate available versions 040 of the medical protocol that is being used with the patient, and/or of one or more medical guidelines from which the medical protocol is derived. The protocol data source 022 may also provide access to the contents, e.g., in textual or structured data form, of the protocol and guideline versions themselves. For example, the protocol data source 022 may provide access to at least the contents of current versions of the guideline and/or protocol, and/or to older versions still in use with patients. The protocol data source 022 may be managed by a medical institution, e.g., by the medical institution where the patient is being treated, managed, or diagnosed, but could also be operated by a third party. The protocol data source 022 may be distributed as well, e.g., guideline versions may be managed and/or stored by a different party than protocol versions. In particular, the version of the protocol that is used with the patient and the further version that it is compared against, may be of different organizations and may accordingly be accessed from different data sources, e.g., operated by these different organizations.

In some embodiments, the protocol data source 022 may be implemented by a version control system, as such as Git, Share Point, or the like. In such cases, the used protocol version may be indicated in patient record 030 by a version number according to the version control system.

In some cases, for example if the system 100 is a PHR system, the protocol data source may provide access to guidelines only, or may only provide access to protocol versions with hospital approval. In such cases, for example, comparisons as described herein may be triggered by a guideline update indicated by the protocol data source 022.

A single data source, such as an EMR database, may provide access to both the patient records 030 and the protocol/guideline versions 040.

Generally, the data interfaces used to access the patient record data source 021 and the protocol data source 022 may take various forms. As illustrated, both sources 021, 022 may be accessed via a common data interface 120. For example, the data interface 120 may be constituted by a data storage interface 120 which may access the data 030, 040 from respective data storages 021, 022, or from a common data storage.

As illustrated, the data storage interface may be an access to remote storage(s) 021, 022, accessed via a computer network 050, for example, a wireless personal area network, an internet, an intranet, a LAN, a WLAN, etc., For instance, data interface 120 may be a personal, local or wide area network interface such as a Bluetooth, Zigbee or Wi-Fi interface or an ethernet or fiberoptic interface, as appropriate for the computer network. For example, the data 030, 040 may be accessed from a remote database using protocols that are known per se, e.g., a remote database access protocol or a REST API, or the like.

The data storage interface 120 can also be a memory interface or a persistent storage interface, e.g., a hard disk or an SSD interface. The data storages 021, 022 may each be an internal data storage of the system 100, such as a hard drive or SSD, but also an external data storage, e.g., a network-accessible data storage. Data 030, 040 may be accessed from different types of data storages, e.g., via a different subsystem of the data interface 120 or via different data interfaces that may each be of a type as is described herein for the data interface 120.

System 100 may comprise the patient record data source 021 and/or the protocol data source 022, but the data sources can also be external to the system.

The system 100 may further comprise a processor subsystem 140 which may be configured to, during operation of the system 100, compare the used version of the medical protocol to a further version of the medical protocol or of the medical guideline that is accessible via the protocol data source, to obtain a change indicator for the used version of the medical protocol. The processor subsystem 140 may be further configured to output the change indicator.

It is not needed in principle to use patient records. In such cases, versions of protocols and/or guidelines may be compared as described herein without reference to any patient record of any particular patient. Also this use of the comparison techniques described herein is envisaged, and many of the advantages of using the provided techniques to compare protocol and/or guidelines versions also apply in this setting. In this case, no patient record data source 021 is needed.

The system 100 may comprise an output interface for outputting the determined change indicator. For example, as also illustrated in Fig. 1, the output interface may be constituted by the data interface 120, with said interface being in these embodiments an input/output (`IO') interface, via which the change indicator may be output, for instance, to the patient record 030. In some embodiments, the output interface may be separate from the data interface 120, but may in general be of a type as described above for the data interface 120. In other embodiments, the system 100 may comprise an output interface to a rendering device, such as a display, a light source, a loudspeaker, a vibration motor, etc., which may be used to generate a sensory perceptible output signal which may be generated based on the determined change indicator. For example, the output signal may be used to automatically alert a user depending on the change indicator. The output interface may also be used to output an aggregate determined over respective change indicators for multiple patients, and/or to notify a user of a mismatch between versions of multiple medical guidelines from which the medical protocol is derived. The output interface may also be an output interface to a separate device operated by the user, such as a smartphone, tablet, or other kind of mobile device. For example, the user may receive an alert as a notification on their mobile device, e.g., via an app that is running on the mobile device and that is notified via the output interface.

The data interface 120 may be used to access additional data, such as parameters of trained models as described herein. This latter data is typically accessed from an internal storage. Generally, a trained model may be parameterized by a set of parameters. For example, the model may be a neural network. Neural networks are also known as artificial neural networks. Examples include deep neural networks and convolutional neural networks. In this case, the set of parameters may comprise weights of nodes of the neural network. For example, the number of layers of the model may be at least 2 or at least 10. The number of parameters may be at least 10 or at least 10000. Architectures for neural networks and other types of machine learnable models may be used as known per se.

In general, each system described in this specification, including but not limited to the system 100 of Fig. 1 may be embodied as, or in, a single device or apparatus, such as a workstation or a server. The device may be an embedded device. The device or apparatus may comprise one or more microprocessors which execute appropriate software. For example, the processor subsystem of the respective system may be embodied by a single Central Processing Unit (CPU), but also by a combination or system of such CPUs and/or other types of processing units. The software may have been downloaded and/or stored in a corresponding memory, e.g., a volatile memory such as RAM or a non-volatile memory such as Flash. Alternatively, the processor subsystem of the respective system may be implemented in the device or apparatus in the form of programmable logic, e.g., as a Field-Programmable Gate Array (FPGA). In general, each functional unit of the respective system may be implemented in the form of a circuit. The respective system may also be implemented in a distributed manner, e.g., involving different devices or apparatuses, such as distributed local or cloud-based servers.

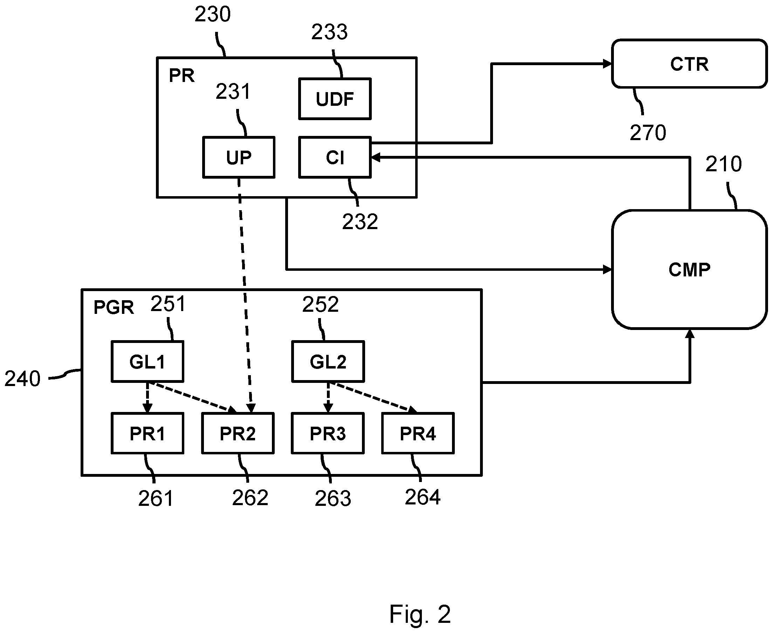

Fig. 2 shows a detailed, yet non-limiting, example of how to determine a change indicator.

Shown in the figure is a patient record **PR**, 230, for example, an electronic medical record (EMR) or a personal health record (PHR). The patient record PR may be accessed via a patient record data source, as discussed in more detail with respect to Fig. 1. The patient record may contain various medical information about the patient as is known per se, e.g., information from one or more medical institutions, information from wearable sensors, etcetera.

The figure further shows a repository **PGR**, 240, of protocols and/or guidelines, for example, a local hospital protocol database. The repository may be accessible via a protocol data source, as discussed in more detail with respect to Fig. 1. The repository **PGR** may indicate available versions of medical protocols for one or more medical conditions, and/or of medical guidelines from which these medical protocols are derived. Although shown as a single unit in the figure, the repository **PGR** may be implemented in a distributed fashion, e.g., where a medical institution provides access to their own protocols and one or more third parties provide access to the underlying guidelines. A medical protocol is also referred to herein as a local guideline, or a care pathway, and can in particular be a medical treatment protocol. The protocol is typically specific to particular medical institution.

As an example, the figure shows four different versions **PR1**, 261; **PR2**, 262; **PR3**, 263; and **PR4**, 264, of a medical protocol for a certain medical condition. The figure also shows two versions **GL1,** 251; **GL2**, 252 of a medical guideline from which the medical protocol is derived. It is common that multiple protocol versions are derived from the same guideline version; e.g., in the figure, protocol versions **PR1**, **PR2** are derived from guideline version **GL1** and protocol version **PR3**, **PR4** are derived from guideline version **GL2**. The repository may provide access at least to the latest versions of the guideline and/or protocol, and/or to version of the protocol that are still currently being applied. As an illustration, a typical guideline may be updated a few times per year, e.g., at most or at least 2 times or at most or at least 8 times. Protocols, being local and easier and faster to update, can be updated more often depending on the preferences of the practicing physicians, e.g., at least bimonthly or at least monthly.

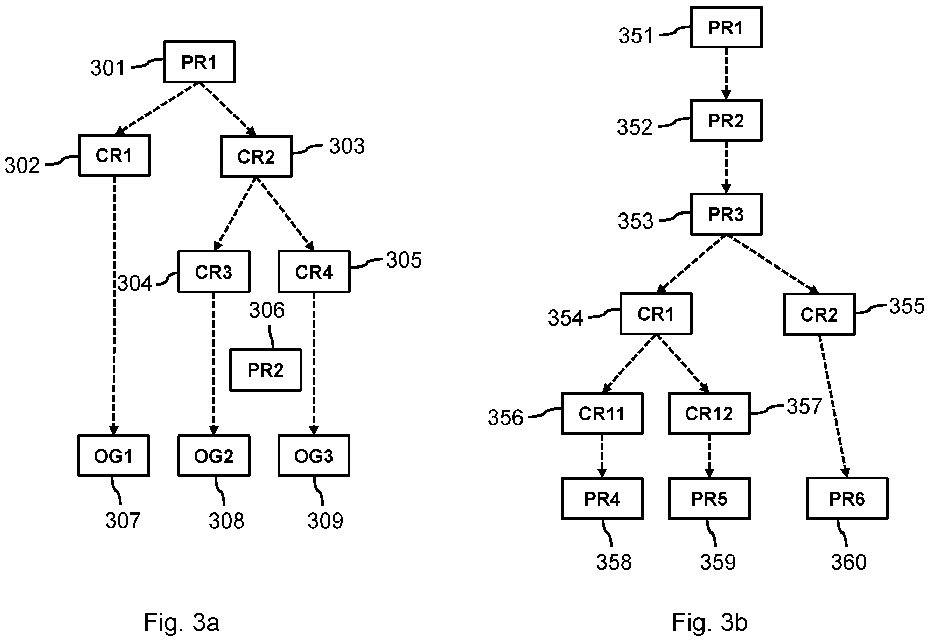

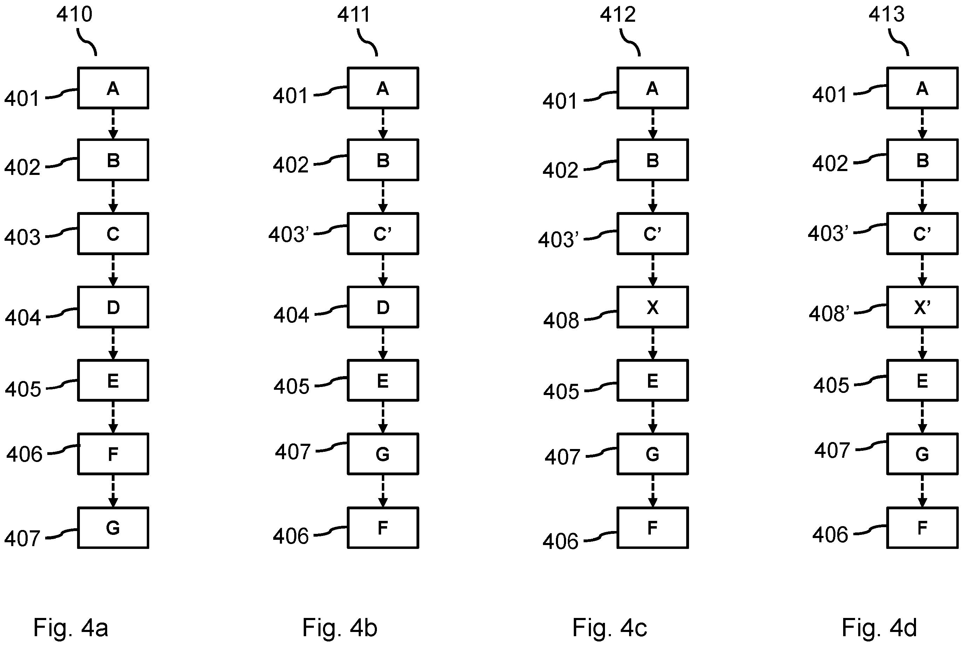

In many cases, the repository **PGR** provides access to the contents of the protocols and/or guidelines as well as to information about their versions. This is not strictly needed, however; for example, in some embodiments, only storing change information is sufficient. Typically, protocols and guidelines have a decision tree structure, meaning they have specific steps, where a decision may be taken at a step, resulting in a follow-up step. A protocol step can comprise performing one or more actions such as applying a standard operating procedure. Examples are discussed with respect to Figs. 3 and 4.

Protocols and guidelines can be stored in the repository **PGR** in various ways. For example, as is usually the case today, protocols and/or guidelines may be stored as textual data, e.g., as a text documents such as a PDF document. Such documents may be stored in hospital database, for example an Electronic Medical Record (EMR) system, using already available solutions for version control. It is also possible, and preferred from a computational efficiency point of view, to store protocols and/or guidelines in a way that reflects their decision tree structure, in particular, as structured data, or as a rule set. Also this is known per se, e.g., see A. Pomares Quimbaya et al., "An Executable Knowledge Base for Clinical Practice Guideline Rules", https://doi.org/10.1016/j.protcy.2014.10.164; or from T. Knape et al., "A UML Approach to Process Modelling of Clinical Practice Guidelines for Enactment", DOI: 10.3233/978-1-60750-939-4-635. Other known examples of accessing protocol data as structured data come from the field of protocol guidance, which are used while accessing and applying protocols in practice. Such applications generate data on how protocols are used, such as databases, logs, and/or annotations, which may be used as protocol data herein.

As illustrated in the figure, the patient record **PR** may indicate a version **UP**, 231, of a medical protocol used with the patient. This protocol version is also referred to herein as the "used protocol". For example, the patient record **PR** may comprise an identifier by which the used protocol **UP** can be retrieved from repository **PGR**, and/or the patient record **PR** may contain the contents of the used protocol, e.g., in textual or structured data form. In this example, the used protocol **UP** refers to protocol **PR2** derived from guideline **GL1**.

The figure further illustrates a comparison operation **CMP**, 210. In this operation, the used version **UP** of the medical protocol may be compared to a further version of the medical protocol **PRi** or of the medical guideline **GLj** from the repository **PGR**. The comparison may result in a change indicator **CI**, 232 for the used version of the medical protocol. As illustrated in the figure, the change indicator **CI** can be output by including the change indicator **CI** in the patient record **PR** for future manual and/or automatic processing. For example, the used version **UP** may be compared to the most recent guideline **GL2** and/or the most recent protocol **PR4**, e.g., triggered by addition of the guideline or protocol, or by finding in a periodic check that the used version **UP** or the guideline **GL1** that it is derived from, or the last version of the guideline or protocol that the used version was compared against, is no longer the most recent. The used version **UP** can be compared to multiple protocols **PRi** and/or guidelines **GLi**, resulting in multiple respective change indicators. Comparison operation **CMP** may for example also compute a difference indicator between the used protocol and a protocol and/or guideline. Such a difference indicator may be used to evaluate a relevance of changes as also described elsewhere.

Generally, the comparison **CMP**, e.g., identification of changes in protocols and/or change feature calculation, may not require access to patient data from the patient record **PR**, other than the identification of the used protocol **UP.** In some embodiments, the physician may be informed, by means of the change indicator **CI,** of the changes applicable to the patient. The physician may determine if changes are relevant for the patient. As also described elsewhere, it is also possible to, using change information as input, access patient data from the patient record **PR**, associated with the change features, and analyze the patient data to output, as part of the change indicator **CI**, applicability data providing an estimation of the applicability (in other words: relevance) of changes to the patient. This approach can be useful for applications that empower patients to search for new options such as new trials, and to initiate contact to their physicians.

In some embodiments, the comparison **CMP** does not use the contents of the protocols or guidelines that are being compared. Such a comparison **CMP** may for instance be based on the timeframe in which protocol updates occur, e.g., by measuring a number of protocol updates in a predefined preceding period of e.g. at most or at least 1 month or at most or at least six months, or another timing characteristic of changes.

In other embodiments, the comparison **CMP** does use the contents of the protocols and/or guidelines. Such a comparison may be based on the subdivision of protocols and guidelines into multiple steps, also referred to herein as procedures. A procedure may comprise an action (e.g., a standard operating procedure, or SOP) and optionally a decision to be taken based on the outcome of the action. The comparison **CMP** may comprise determining a comparison output for a given procedure, indicating a change between the compared versions with respect to application of the given procedure. In particular, SOPs are well defined and have clear start and end definitions. Hence, they can be evaluated independently from each other.

In some embodiments, the protocol/guideline data **PRi**, **GLi** may be stored in a structured format organized according to the procedures. Where this is not the case, the comparison **CMP** may comprise the use of natural language processing (NLP) to identify segments corresponding to respective procedures, and/or may use metadata (e.g., additional notes or explanation; timestamps; or links to guidelines, patient records, or healthcare providers) and/or formatting (e.g., use of headings and the like) to identify segments corresponding to respective procedures.

It is noted that the comparison **CMP** is done between different versions of the same protocol, and not between different protocols. This means that the protocol may be the same in terms of the type of disease, patient condition, or healthcare-related action being addressed by the protocol. In particular, the protocols may have the same name. This means that most of the procedures in the compared protocols are likely to be the same or similar, because only few of the procedures are likely to change at a given time or change across healthcare providers. Accordingly, it may be feasible to match procedures between the versions, or at least to match procedures with a change. On the other hand, a failure to attribute a change to a particular procedure, may indicate a significant change in the protocol or guideline that needs to be notified, e.g. a special change indicator **CI** indicating a failure to match may be output.

In the example below, the used version of the protocol **UP** may be referred to as "protocol 0" and the version of the protocol or guideline that the used version is compared against, may be referred to as "protocol 1".

A comparison output for a given procedure may indicate no change; a change; an addition; a removal; or a rearrangement. This may be encoded for example as follows:
0: A (no change)
1: B1 (procedure changed)
2: B2 (new procedure added)
3: B3 (deleted procedure)
4: B4 (order, or relative position of procedure changed)
5: B5 (new protocol)

The comparison outputs may be aggregated into an overall output to be included in the change indicator **CI**.

Generally, the change indicator **CI** may comprise one or more change features. These may generally be determined using text analysis tools that are available per se. Further, a timestamp may be associated with the change indicator **CI** corresponding to the time when the difference is recorded or detected. Considering that comparison may be performed immediately or relatively soon after a new protocol becomes applicable and is stored in the repository **PGR**, the timestamp many indicate an accurate time of parts of protocol 0 being changed. Also associated with the change indicator **CI** may be information about the used protocol **UP** and/or the further protocol(s)/guideline(s) that the used protocol was compared against; this information may be used to determine later if the change indicator is to be updated.

As an example, the change indicator **CI** may include one or more codes representing changes made to the set of procedures of the protocol, e.g.:
A. no change
B. change
B1. protocol step changed
B2. new protocol step added
B3. a protocol step removed
B4. order of steps changed
B5. steps could not be matched between protocol 0 and protocol 1 -- e.g., old protocol is replaced by a new protocol with significant changes, e.g.: making MRI part of the prostate screening.

It is also possible to use other types of aggregate. For example, the change indicator **CI** may comprise a binary protocol change indicator showing if protocol 0 is different than protocol 1.

One or more change features of the change indicator **CI** may represent a count and/or timing of changes, e.g., based on comparison outputs per procedure. This is further illustrated with respect to Figs. 4a-4b.

Various other change features can also be calculated by evaluating the content and meaning of changes. In particular, the comparison can be based on extraction of medical terms, such as name of parameters, medications, lab values, procedures. In such case, a numerical value or text description linked in the text to these medical terms, features, parameters, etc. may be used for the comparison. Such values may be calculated for example by using keyword search or by applying respective feature extractors, for example, specifically trained machine learning algorithms such as a natural language engine that is specifically trained to evaluate the protocol (e.g., ablation protocol) in question.

For example, the change indicator **CI** may include change features based on one or more of the following extracted features:
- a vital parameter value mentioned in a protocol step
- a vital parameter type mentioned in the protocol step
- a measurement mentioned in a protocol step
- a drug mentioned in a protocol step
- a decision boundaries mentioned in a protocol step
- a measurement type mentioned in the protocol step;
- a tool or a device mentioned in the protocol step;
- a physical location mentioned in the protocol step;
- a body location mentioned in the protocol step;
- an algorithm or algorithm output mentioned in the protocol step;
- a time related indicator, such as a duration or time of day, mentioned in the protocol step;
- an amount-related factor (such as ml, liter) mentioned in the protocol step.

For example, the features may be extracted from protocol 0 and protocol 1, with their difference being included in the change indicator **CI**. An alternative approach is to first compare protocol 0 and protocol 1 to determine a difference, and then apply the feature extractor to the difference. In any case, it is noted that calculation of such features does not require access to patient data.

Generally, since protocol/guideline data in repository **PGR** is typically text-based, performing the comparison may be performed in a computationally efficient way. Language understanding techniques may be used to interpret information about respective procedures; keyword search may be used to look up e.g. a parameter for a specific diagnostic step. Changes in protocols/guidelines may be identified in an efficient manner, for example, using document comparison software that is known per se, or by applying natural language understanding. Also feature extraction can be efficient. The computational power of a personal computer typically suffices.

In some embodiments, the change indicator **CI** may be based on recommendation strength indicators for respective procedures. Generally, a detected difference between the used protocol **UP** and the protocol or guideline that it is compared against, may be related to a change in the corresponding guideline: e.g., the guideline that is being compared against, or the guideline used to derive the protocol that is being compared against. The recommendation strength may indicate a strength of the recommendation for change in the guideline and may be derived from e.g., NCCN publications, panel discission notes and votes, clinical trial results leading to change, or healthcare provider information (e.g., a reputation of a hospital making a change). The recommendation strength may be used to rank the features, e.g., to compute an aggregate change indicator that assigns higher weight to higher-ranked (more strongly recommended) features and lower weight to lower-ranked (less strongly recommended) features. As an alternative to manually deriving recommendation strength values from publications, the recommendation strength may also be determined automatically by using a machine learning algorithm (e.g., natural language processing) to analyze guideline text and/or additional sources, e.g., publications describing a corresponding clinical trial that is the reason for change. It is noted that this can be done again without accessing the patient data.

It is not necessary to derive change features based on a per-procedure subdivision of the protocols and/or guidelines being compared. An alternative is to apply a trained feature extractor to data (e.g., textual data) representing full protocols/guidelines to be compared, or to a difference between the full protocols/guidelines to be compared, to obtain an overall feature extractor output. Features of this overall feature extraction may be included in or otherwise used to determine the overall change indicator CI. In other words, a data mining approach may be used where inputs are first processed to extract features, and further processing is done on the extracted features.

In some embodiments, an applicability of detected changes may be determined based on the patient data comprised in the patient record **PR** of the patient. The applicability may be taken into account when determining the change indicator, e.g., only changes to those procedure detected to be applicable, or potentially applicable, to the patient may be taken into account; or a separate change feature estimating the effect of changes on the patient can be calculated and included in the change indicator **CI**.

For example, a machine learning algorithm can evaluate the effect of the changes on the patient. The machine learning algorithm may be applied to the patient data and to data representing the protocols/guidelines being compared and may output an estimated effects of the new protocol/guideline, or may output differences of the new protocol/guideline with respect to the initial protocol **UP** being used with the patient. These set of features require processing of patient data.

Upon being output by the comparison operation **CMP** in the patient record **PR**, the change indicator **CI** may be automatically processed by one or more user-defined functions **UDF**, 233, defined in the patient record **PR**. In particular, a user-defined function may be defined by a physician, in which case it is also referred to as physician-defined function, rule, or criterion. A user-defined function **UDF** may use one or more computed change features (of a particular procedure, or of the protocol/guideline as a whole), and may define conditions under which a further action, such as informing the physician or another user, is taken. Thus, some changes (e.g., addition of new drug) to the protocol may not result in any actions, while other changes (e.g., change in the recommended dosage for the medication used by the patient) may lead to an action according to the user-defined function **UDF**. There may be multiple user-defined functions, for example, one function to alert a physician, healthcare provider, or institution; a different function to alert the patient; and/or a different function to alert a caregiver. The user-defined function(s) **UDF** may be defined specifically for a particular patient, but can also be configured for a group of patients or all patients of a certain patient data source, for example.

Two illustrative examples of user-defined functions and associated actions are as follows:
f1 : if total change count > 4, or change_rate>1/3: send patient PHR to physician for evaluation
f2: if change_rate> 2/3 inform patient to contact physician, and inform physician to evaluate patient PHR

A user-defined functions can for example be defined by a healthcare provider as a part of treatment monitoring and follow up.

In some embodiments, change indicators **CI** may be determined for multiple respective patient records **PR** of multiple respective patients, and an aggregate may be computed over the respective change indicators **CI**. For example, the computation of such an aggregate may be triggered when a protocol update impacts the treatment or diagnosis of a particular patient, and the aggregate may be output along with the change indicator **CI** for the patient. This way, a physician may directly learn information about the further impact of the protocol change, e.g., a total number of affected patients. This may help the physician and/or hospital to plan accordingly. By determining the aggregate, the effects of updates on multiple patients can be monitored at once.

In some embodiments, a mismatch may be determined between versions of multiple medical guidelines **GL1**, **GL2** from which the used medical protocol **UP** is derived. Upon determining such a mismatch, a user may be notified. As is known per se, in some instances, there may be inconsistency between guidelines. It is useful to identify such inconsistencies, especially if a hospital protocol is based on multiple guidelines, since conflicting recommendations can impact the standard of care. The mismatch can be identified using the protocol change comparison mechanism **CMP** described herein. In case a conflict is detected, the treating physician and/or a hospital/appropriate authority level expert panel may be alerted to avoid poor quality/nonstandard patient management.

In some embodiments, a clinical trial recommendation operation **CTR**, 270, may be applied to a determined change indicator **CI** to determine if a clinical trial recommendation applies. In situations where the patient does not benefit from the standard of care, or the guideline recommended treatment has a poor prognosis, guidelines **GLi** often recommend enrolling in a clinical trial. However, information about specific trials is typically absent from the guidelines. The provided techniques allow a systematic way for the treating medical professional, patient, or other stakeholder, to become aware of a suitable clinical trial. This may be based on the insight that guideline changes typically happen because a new finding becomes available from clinical trial. By keeping track of which clinical trials triggered a guideline and consequently a protocol change, advice about potentially relevant clinical trials may be derived.

In particular, a guideline **GLi**, or a protocol **PRi** derived from it, may indicate a clinical trial recommendation for a certain patient trajectory. Using patient data from the patient record **PR**, a treatment trajectory of the patient according to the protocol or guideline may be derived, and it may be determined if the clinical trial recommendation applies.

In more detail, patient data may be used to map a patient's prognosis trajectory in a disease management workflow and identify guideline recommendations for clinical trial enrolment. The patient data used may include patient disease stage (e.g., TNM), grade, demographic information (age, gender etc.), vital parameters, and/or comorbidities. Further, information about current management and place in the protocol may be used. Based on this information, the patient may be localized in the guideline/protocol; the prognosis trajectory may be estimated; and it may be decided if enrolling in a clinical trial is recommended.

Alternatively, the clinical trial recommender **CTR** may operate without using patient data and protocol/guideline contents. In particular, the trial recommender may use the frequency of updates to the guidelines/protocols. Namely, as the inventors realized, the higher the update frequency, the higher the likelihood that a clinical trial is running or that the output of the clinical trial is included in the guidelines or protocols. Accordingly it may be possible, without using patient data, to indicate the possibility of a trial. For example, a clinician can then asses the patient data and determine the applicability to the patient.

The clinical trial recommender **CTR** may further access a clinical trial registry and automatically search for a clinical trial matching the clinical trial recommendation and/or the patient data. In case it is observed that the patient may benefit from enrolling in a clinical trial, a search may be performed, e.g., in a geographically appropriate clinical trial registry. A found clinical trial may be output as a recommendation, e.g., to the treating physician and/or the patient. For example, a clinical trial registry such as ClinicalTrials.gov (in the USA) or https://ctri.icmr.org.in/ (in India) may be used.

The data in the patient record may be monitored in the sense that the comparison **CMP**, and any subsequent updating of the change indicator **CI** and/or any subsequent alerting, may be triggered automatically and not in response to an explicit user command that needs to be given for each comparison. For example, the comparison **CMP** may be performed regularly and/or in response to a trigger. The regular comparison may, for a given patient, for example be at most or at least once every day or at most or at least every month. The trigger may for example be an update to the protocol/guideline registry **PGR** relevant to the treatment or diagnosis that the patient is receiving, and/or an update to the patient record **PR** itself. The comparison can also be part of a real-time protocol guidance to a clinician. In any case, the comparison may comprise a first check if the change indicator **CI** is to be recomputed; e.g., the change indicator **CI** may indicate versions of the protocols/guidelines that were used to compute it, based on which the comparison operation **CMP** may determine whether an update is needed, e.g., because the used protocol **UP** has changed, or a new protocol or guideline is available. If the update is needed, the change indicator may be determined as described herein. The change indicator may in any case be recomputed at a regular basis e.g., to account for changes in the patient data, if using, that may also affect the change indicator **CI**.

It is noted that, in principle, the change indicator **CI** does not need to be associated with any particular patient record, and can also be used to compare protocol and/or guideline versions without regard to any particular patient. In this case, no patient data may be needed at all. One application of this can be to compare protocols among different healthcare providers. For example, a group of protocols may be defined that spans multiple healthcare providers, e.g., by annotation with a common label. The protocols may be related e.g. in that they are used to treat same conditions and/or are derived from the same guideline. This group information can be used to track changes in any of the protocols. For example, a change indicator at one healthcare provider, e.g., indicating that there are significant changes in protocol2 used by provider2, may be used to alert another healthcare provider concerning their respective protocol of the same group (e.g. protocol1 used by provider1), e.g., informing the other healthcare providers of change features (e.g. frequency, type, number), and/or of changes (e.g. content) in the protocols of provider2. Such information may trigger the informed providers to re-assess their protocols.

Figs. 3a-3b show examples of medical guidelines. These examples illustrate that guidelines can typically be fitted into a decision tree structure. In particular, the figures illustrate that a single procedure may involve multiple decisions. For example, for cancer treatment guidelines, it is in fact typical that multiple options are provided at tree nodes.

In particular, Fig. 3a is a schematic illustration of a guideline, based on the EAU-EANM-ESTRO-ESUR-ISUP-SIOG Guidelines on Prostate Cancer as current in March 2021. The figure shows a decision tree for health status screening for men > 70 years.

Procedure **PR1**, 301, represents screening by G8 and mini-COG.

Criterion **CR1**, 302 of procedure **PR1** represents a decision whether G8 score > 14/17, in which case no geriatric evaluation is needed, and the patient is assigned to outcome group **OG1,** 307, fit.

Criterion **CR2**, 303 of procedure **PR1** represents a decision whether G8 score ≤ 14/17, in which case a full geriatric evaluation is mandatory.

Further criterion **CR3**, 304 represents a decision whether abnormal ADL is 1 or 2; weight loss is 5-10%; and comorbidities are CISR-G grades 1-2. According to this decision the patient is assigned to outcome group **OG2**, 308, vulnerable.

Further criterion **CR4**, 305 represents a decision whether abnormal ADL is > 2; weight loss is > 10%; and comorbidities are CISR-G grades 3-4. According to this decision, the patient is assigned to outcome group **OG3**, 309, frail.

In case of criteria **CR3**, **CR4**, a procedure **PR2**, 306, Geriatric assessment then geriatric intervention, may be performed.

Fig. 3b is a further example of a guideline, based on the guideline SYSTEMIC THERAPY FOR M1 CRPC from J.L. Mohler et.al., "Prostate cancer, version 2.2019", JNCCN, Volume 17, Number 5, May 2019.

Procedure **PR1**, 351, represents CRPC, conventional imaging studies positive for metastases.

Procedure **PR2**, 352, represents several procedural steps: consider metastatic lesion biopsy; consider tumor testing for MSI-H or dMMR; consider genetic counselling and germline testing for homologous recombination gene mutations.

Procedure **PR3**, 353, represents the procedural step to continue ADT to maintain castrate levels of serum testosterone (<50 ng/dL); and provides several additional treatment options: bone antiresorptive therapy with denosumab (category 1, preferred) or zoledronic acid if bone metastases present; immunotherapy with sipuleucel-T (category 1); palliative RT for painful bone metastases; and best supportive care.

Criterion **CR1**, 354, corresponding to procedure **PR3** represents adenocarcinoma.

Further criterion **CR11**, 355, corresponding to procedure **PR3** represents no visceral metastases and refers to procedure **PR4**, 358, coded as PROS-17.

Further criterion **CR12**, 357, corresponding to procedure **PR3** represents visceral metastases and refers to procedure **PR5**, 359, coded as PROS-18.

Criterion **CR2**, 355, corresponding to procedure **PR3** represents small cell/neuroendocrine prostate cancer and refers to procedure **PR6**, 360, providing first and subsequent therapy options: chemotherapy (cisplatin/etoposide, carboplatin/etoposide, docetaxel/carboplatin); clinical trial; and best supportive care.

Figs. 4a-4d show a detailed, yet non-limiting, example of determining change features. The respective figures show four different versions 410-413 of a protocol. The figures illustrate a number of changes that can be made to such protocols and how they can affect some of the change features discussed with respect to Fig. 2.

This figure demonstrates several change features based on a count and/or timing of changes at the procedure level. Such change features may be determined by determining a comparison output for a given procedure, in other words, by comparing the occurrence of the given procedure for the protocol versions being compared. The comparison output may indicate a change between the versions with respect to application of the given procedure, e.g.: no change; a change; an addition; a removal; or a rearrangement. The comparison outputs for respective procedures may be aggregated into an overall output, e.g., included as a change feature in the determined change indicator. Interestingly, computation of these change features may be performed without using patient data.

In particular, the change features illustrated with respect to these figures are:
- count of changes: the number of unique changes (change/addition/removal/ rearrangement);
- rate of changes: number of changes per time period.

Show in the figure is a version "Protocol 0", 410, of the protocol. The protocol comprises steps (e.g., standard operating procedures) **A**, 401; **B**, 402; **C**, 403; **D**, 404; **E**, 405; **F**, 406, and **G**, 407 being executed in sequence. In another version "Protocol 1", 411, of the protocol, Protocol 0 is changed by altering step **C** to step **C'**, 403', and by changing the order of steps **F** and **G**. In another version "Protocol 2", 412, of the protocol, Protocol 1 is changed by removing step D and adding step X, 408. In another version "Protocol 3", 413, of the protocol, Protocol 2 is changed by modifying step X to step X', 408'.

These protocol versions, and the computation of the change features, may be summarized in the following table:

### Protocol Protocol steps Time of Chnge Tot ch Change rate version update count count

Protocol 0 A B C D E F G January 0 0 0
Protocol 1 A B C' D E G F April 2 2 2/3
Protocol 2 A B C' X E G F July 2 4 2/3
Protocol 3 A B C'X'E G F October 1 5 1/3

For example, in protocol 411, two changes are made compared to protocol 410: step **C** is changed to step **C'** and steps **F** and **G** are rearranged. So the change count is 2. Dividing this number of changed by the time between protocols 410 and 411, namely, three months, provides the change rate. Also the total change count is shown as an illustration. As the skilled person understands, many variations of the above change features for representing a count and/or timing of changes are possible, e.g., different types of changes can be weighted differently; the change rate can for example represent a number of times the overall protocol is updated, or a number of changes to individual protocol steps, e.g. over a predefined time period.

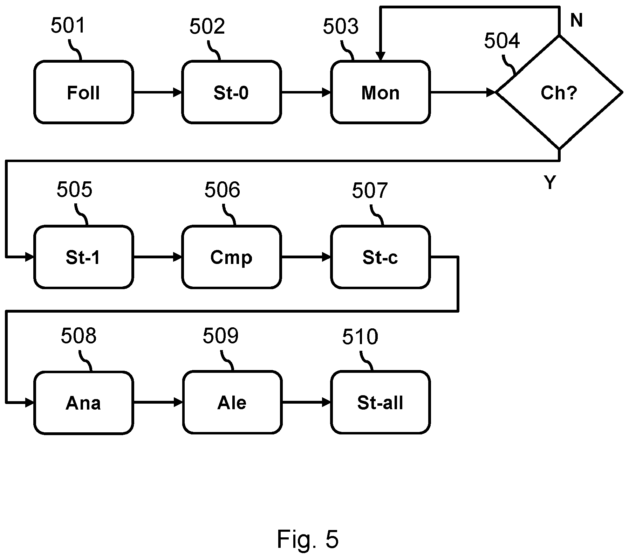

Fig. 5 shows a detailed, yet non-limiting, example of a method of monitoring patient data. This example illustrates determining a change indicator and outputting it to a patient record as well as alerting a user depending on the change indicator.

In a follow operation **Foll**, 501, a certain protocol version "Protocol 0" may be followed with a patient. In a store operation **St-0**, 502, an indication that the protocol version "Protocol 0" is used with the patient, may be stored in a patient record of the patient, e.g., a personal health record.

In a monitoring operation **Mon**, 503, changes in Protocol 0 may be monitored. For example, it may be checked if Protocol 0 is the most recent version of the protocol that is available in a protocol data source. The monitoring **Mon** may comprise deciding if there is a protocol change **Ch?**, 504. If not **N,** the monitoring **Mon** may continue without determining a change indicator If so **Y**, determination of a change indicator may be initiated. Optionally, the new protocol version, e.g., "Protocol 1" may be stored in the patient health record **St-1**, 505, e.g., to record that a comparison with this protocol version is performed. For example, a reference to "Protocol 1" and/or the contents of the protocol version may be stored by operation **St-1.** In a comparison operation **Cmp**, 506, the used version "Protocol 0" of the medical protocol may be compared to the further version "Protocol 1" to obtain a change indicator, e.g., comprising one or more change features. In an operation **St-c**, 507, the change indicator may be stored in the patient record.

In an analysis operation **Ana**, 508, the change indicator, e.g., the change features, may be analyzed to determine whether to alert a user. For example, the alert may be initiated if the change indicator satisfies a predefined condition, e.g., if one or more change features of the change indicator exceed one or more corresponding predefined thresholds, e.g., if a count and/or a frequency of changes exceed thresholds. For example, the analysis operation may be implemented as one or more user-defined functions of the patient record. If it is determined that a user, e.g. the physician and/or patient, is to be alerted, an alert operation **Ale**, 509, may be performed to this end. Finally, an optional storage operation **St-all**, 510, is shown, in which any additional information may be stored in the patient record, e.g., information about the fact that an alert has been performed etc. Storage operation **St-all** may for example be performed automatically or when this option is chosen by a user, e.g., the physician.

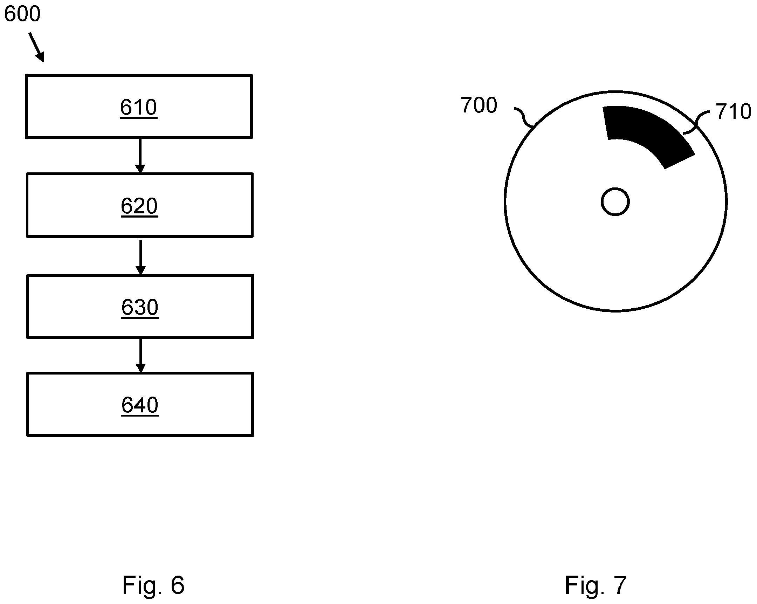

Fig. 6 shows a block-diagram of computer-implemented method 600 of monitoring patient data. The method 600 may correspond to an operation of the system 100 of Fig. 1. However, this is not a limitation, in that the method 600 may also be performed using another system, apparatus or device.

The method 600 may comprise, in an operation titled "ACCESS PATIENT DATA", accessing 610 a patient record data source. The patient record data source may enable to access a patient record of a patient. The patient record may indicate a version of a medical protocol used with the patient, e.g., for treatment, management, or diagnosis.

The method 600 may comprise, in an operation titled "ACCESS PROTOCOL DATA", accessing 620 a protocol data source. The protocol data source may indicate available versions of the medical protocol and/or of a medical guideline from which the medical protocol is derived.

The method 600 may comprise, in an operation titled "COMPARE", comparing 630 the used version of the medical protocol to a further version of the medical protocol or of the medical guideline accessible via the protocol data source to obtain a change indicator for the used version of the medical protocol.

The method 600 may comprise, in an operation titled "OUTPUT", outputting 640 the change indicator.

As also discussed with respect to Fig. 1., the operation 610 of accessing a patient record data source to access patient records is not needed in principle. In such a case, protocol and/or guidelines versions may be compared without reference to any particular patient record. The advantages of the provided comparison techniques still apply.

It will be appreciated that, in general, the operations of method 600 of Fig. 6 may be performed in any suitable order, e.g., consecutively, simultaneously, or a combination thereof, subject to, where applicable, a particular order being necessitated, e.g., by input/output relations.

The method(s) may be implemented on a computer as a computer implemented method, as dedicated hardware, or as a combination of both. As also illustrated in Fig. 7, instructions for the computer, e.g., executable code, may be stored on a computer readable medium 700, e.g., in the form of a series 710 of machine-readable physical marks and/or as a series of elements having different electrical, e.g., magnetic, or optical properties or values. The medium 700 may be transitory or non-transitory. Examples of computer readable mediums include memory devices, optical storage devices, integrated circuits, servers, online software, etc. Fig. 7 shows an optical disc 700. Alternatively, the computer readable medium 700 may comprise data 710 representing a patient record of a patient. The patient record may indicate a version of a medical protocol used with the patient. The patient record may further comprise a change indicator for the used version of the medical protocol, as described herein.

Examples, embodiments or optional features, whether indicated as non-limiting or not, are not to be understood as limiting the invention as claimed.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of' when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A computer-implemented method (600) of monitoring patient data, comprising:
- accessing (610) a patient record data source, wherein the patient record data source enables to access a patient record of a patient, wherein the patient record indicates a version of a medical protocol used with the patient;
- accessing (620) a protocol data source, wherein the protocol data source indicates available versions of the medical protocol and/or of a medical guideline from which the medical protocol is derived;
- comparing (630) the used version of the medical protocol to a further version of the medical protocol or of the medical guideline accessible via the protocol data source to obtain a change indicator for the used version of the medical protocol, and outputting (640) the change indicator.

2. The method (600) of claim 1, comprising outputting the change indicator to the patient record and/or automatically alerting a user depending on the change indicator.

3. The method (600) of claim 2, wherein the patient record comprises a user defined function configured to automatically alert the user.

4. The method (600) of any preceding claim, wherein comparing the used version to the further version comprises determining a count and/or timing of changes.

5. The method (600) of any preceding claim, wherein comparing the used version of the medical protocol to the further version comprises determining a comparison output for a given procedure, wherein the comparison output indicates a change between the used version and the further version with respect to application of the given procedure.

6. The method (600) of claim 5, comprising obtaining recommendation strength indicators for respective procedures, wherein the change indicator is based on the recommendation strength indicators.

7. The method (600) of claim 5 or 6, further comprising accessing patient data of the patient; using the patient data to determine applicability data indicating an applicability of the change to the patient; and basing the comparison output on the applicability data.

8. The method (600) of any one of claims 5-7, further comprising applying a feature extractor to data about the given procedure comprised in the used version and/or in the further version and/or in a difference between the used version and the further version to obtain a feature extractor output for the given procedure, and using the feature extractor output to determine the change indicator.

9. The method (600) of any one of claims 1-4, comprising applying a feature extractor to data representing the used version and/or the further version and/or a difference between the used version and the further version to obtain an overall feature extractor output, and using the overall feature extractor output to determine the change indicator.

10. The method (600) of any preceding claim, further comprising determining based on the comparison if a clinical trial recommendation applies, and if so, outputting the clinical trial recommendation.

11. The method (600) of any preceding claim, wherein the used version and the further version are of different medical institutions and/or from different protocol data sources.

12. The method (600) of any preceding claim, comprising determining respective change indicators with respect to a further version for multiple patients; computing an aggregate over the respective change indicators; and outputting the aggregate.

13. The method (600) of any preceding claim, wherein the comparing comprises determining a mismatch between versions of multiple medical guidelines from which the medical protocol is derived, and notifying a user of the mismatch.

14. A medical data system (100) for monitoring patient data, comprising:
- a data interface (120) for accessing a patient record data source, wherein the patient record data source enables to access a patient record (030) of a patient, wherein the patient record indicates a version of a medical protocol used with the patient;
- a data interface (120) for accessing a protocol data source, wherein the protocol data source indicates available versions (040) of the medical protocol and/or of a medical guideline from which the medical protocol is derived;
- a processor subsystem (140) configured to compare the used version of the medical protocol to a further version of the medical protocol or of the medical guideline accessible via the protocol data source to obtain a change indicator for the used version of the medical protocol, and to outputting the change indicator.

15. A transitory or non-transitory computer-readable medium (700) comprising data (710) representing:
- instructions which, when executed by a processor system, cause the processor system to perform the computer-implemented method according to any one of claims 1 to 13, and/or
- a patient record of a patient, wherein the patient record indicates a version of a medical protocol used with the patient, and wherein the patient record further comprises a change indicator for the used version of the medical protocol obtained by comparing the used version of the medical protocol to a further version of the medical protocol or of a medical guideline from which the medical protocol is derived.
